# EUROPEAN PATENT SPECIFICATION

(11) **EP 3 510 948 B1**
(45) Date of publication and mention of the grant of the patent: **26.02.2020**
(21) Application number: 18151662.6
(22) Date of filing: 15.01.2018
(51) Int. Cl.: A61B 17/16

(54) **REAMER WITH VARIABLE SHARPNESS**
REIBAHLE MIT VARIABLEN SCHÄRFEN
ALÉSOIR À NETTETÉ VARIABLE

(43) Date of publication of application: 17.07.2019
(73) Proprietor: Arthrex Inc, Naples, FL 34108-1945 (US)
(72) Inventor: Michel, Gerlinde, 80999 Munich (DE); Naudin, Stéphane, 82152 Krailling (DE)
(74) Representative: Lohr, Jöstingmeier & Partner

(56) References cited:
- EP-A2- 2 561 822
- GB-A- 2 406 278
- US-A1- 2015 265 288
- US-A1- 2017 014 141

## Description

### Field of the invention

The present invention relates to the field of surgical tools and, more particularly, to a reamer with specific surface blade configuration to improve drilling capabilities during surgery. The closest prior art is document GB 2406278 A, which discloses the preamble of claim 1.

### Description of the related art

During surgical procedures, it is often necessary to approach a specific anatomical location precisely and directly, to avoid unwanted surgical trauma. For example, orthopedic surgery and related trauma care requires bone reaming to allow for implants. These procedures may include hip replacement, knee replacement, and shoulder replacement. It is important to minimize damage and protect surrounding soft tissue during surgery.

Bone reamers usually have a good reaming performance in the peripheral area, but poor performance in the center. As a result exceeding reaming force is needed to cut through the initial cortical bone (the most exterior hard part of the bone) portion leading to overheating of the bone and of the surrounding area. This causes bone necrosis which is deleterious for good bone ingrowth after surgery. Also during reaming of a bone, such as a humeral head with a conventional reamer, an uncontrolled overshoot normally occurs once the hard cortical bone has been penetrated.

A system and a method for reaming bones is disclosed in EP 2131746 B2. According to this document, conventional reamers can cut cortical bone initially but can quickly dull after a single use, or at best a few uses. Once the reamer has dull cutting areas, it reduces the efficiency of bone cutting and in addition generates sufficient friction/heat to damage or kill the surrounding bone. Here, the problem is solved by employing a hollow reamer for medical applications having a disposable hollow cutter assembly which can be attached to a reusable shaft portion, whereby after one use of the reamer, a new hollow cutter assembly is utilized and the used hollow cutter assembly is discarded. To be noted here is the economical and material wastage occurring after every medical procedure. The reamer is simply disconnected from the shaft and discarded. The reamer as such is not improved but just replaced by a new one.

A further prior art document EP 0508710 discloses a reamer with a rotatable body, a drill tip including tip edges, reaming edges joined to the tip edges and extending from said tip edges to form a tunnel. When said body is rotated in the bone, flute surfaces are disposed between said tip edges for evacuating the bone. This device solves the problem of more efficient cutting and less heat by the angle of the bore blades. However, the blades are uniformly constant sharp over their entire lengths. As such, after a couple of uses these reamers become dull and less efficient at least in the areas having most contact with the bone to the extent that they need to be replaced earlier, although the majority of the blades may not have reached their end of use live.

After long scientific research it has been concluded that none of the prior art documents satisfies the need for a reamer which is easy and precise to use without overshooting and deforming the hole when penetrating the cortical bone part, protects the surrounding soft tissue, cuts the bone at a constant speed, and is reusable and hence more economically viable.

### Summary of the invention

The problem to be solved by the invention is to provide a reamer which solves the problem of overshooting and deforming the hole in the process with aggravating consequences for the implant to be fitted to the bone. A further aspect relates to providing a better and more precise cutting tool of a bone without inflicting further injury to the bone by overheating and overshooting the mark.

The solution of the problem is described in the independent claim, which defines the invention. The dependent claims relate to further improvements of the invention.

In an embodiment, a reamer provides variable sharpness. The sharpness decreases with increasing radius from the reamer axis. Therefore, close to the axis is the highest sharpness. Herein a higher sharpness preferably relates to the ability of removing more material and/or removing material with a lower force applied to the reamer.

The result is an embodiment that can reduce poor performance of bone reaming and allows for a constant pressure to be applied to the reamer throughout the entire reaming procedure.

In a preferred embodiment, a reamer comprises a flat body preferably having a cylindrical contour extending in a single plane and having a thickness between a proximal reamer area being the front face of the reamer and a distal reamer area being the back of the reamer. The reamer preferably has a center connection ring which may be attached to a reamer shaft or a retro pin, and preferably has at least one first blade wing and at least one second blade wing preferably being equal in shape and preferably extending in opposite directions in said single plane. The retro pin has means for connecting to the reamer, it preferably has an elongated shaft and may have a stop plate at its distal end. The retro pin may extend through the connection ring of the flat reamer body from the distal end towards the proximal direction. The at least one first blade wing and the at least one second blade wing may have a first side and an opposing second side. The first side and second side of the at least one first blade wing and the at least one second blade wing are preferably parallel to each other and are connected to the connection ring on one side and to a depth limit stop on the opposite side. Preferably, the first side and second side of the at least one first blade wing and the at least one second blade wing form a triangular form, preferably an isosceles triangular form, with an acute angle connected to the center connection ring and the hypotenuse opposite to the center connection ring connected to the depth limit stop. The depth limit stop may be circular and may be offset in the distal direction compared to the blade wings. The depth limit stop may act as a hole depth limit stop. The first side and second side of the at least one first blade wing and the at least one second blade wing may be slightly arcuated in a rotational direction.

The blades wings may have first cutting areas and second cutting areas located at the proximal reamer area. Preferably, the blades may be located on the forward rotating edge on the proximal area of the blade wings. Also preferably, the blades are separated or divided in sections by recesses. These recesses may be intermittently interrupting the continuation of the blades.

In a further embodiment, the depth limit stop may be in the form of an arc segment of a circle. The arc segments of the depth limit stops are offset in the distal direction compared to the blade wings. The arc segments depth limit stop act as a hole depth limit stop. When in rotation around the vertical shaft axis of the reamer, the arc segments stop of the blades follow an arch path back where they started, completing a full circle. This full circle is the maximum radius of the reamer.

Preferably, the blades have different dimensions depending of the anatomical necessity of the joint of a bone to be reamed. For example for a larger bone joint of a male, the blades may be larger and thicker, creating a larger radius and a deeper hole, while for a smaller bone joint for a female, smaller blade wings with reduced radius and thickness may be needed. Preferable the reamer protrudes no further than the circular or arc segment depth limit stop.

The blade wings may have different thickness preferably from 0,1 mm - 200 mm, more preferable from 0,1 mm - 20 mm and even more preferably from 0,1 mm - 10 mm. The radius of the flat reamer may be from 10 mm - 100 mm, preferably from 20 mm -70 mm, and more preferably from 25 mm -50 mm.

The at least one first blade wing and the at least one second blade wing may have cutting areas with variable sharpness. Preferably, the sharpness may vary within each cutting area blade and between the cutting areas. Preferably, the closer the cutting areas to the vertical reamer shaft axis the sharper the blades. Preferably the sharpness of the reamer cutting areas decreases with the distance away from the vertical shaft axis. The highest sharpness is preferably on or adjacent to the central connection ring.

Preferably, the cutting areas may be in the form of a single blade situated on the proximal edge of the slightly arched blade wings. Even more preferably, the blade may be situated on the proximal edge of the slightly arched blade wings and may be intermittently interrupted. This would give it the appearance of many teeth next to each other similar to the teeth of a rake. Objectively, these are many small blades next to each other which are placed on the proximal edge of the slightly arched blade wings facing the rotation direction and separated by channels or recesses between each other. Preferably, the cutting blades are not identical. Preferably, the first cutting area blade rotates in the same radius of the second blade's second recess. Preferably, the opposite is the case with the cutting areas of the second blade and the first recesses of the first blade. In other words, the radial distance of the cutting areas of one blade wing equals the radial distance of the recesses on the opposite blade wing. Preferably, the radial width of the cutting areas of one blade wing is larger than the radial width of the recesses on the opposite blade wing. This may ensure, that all bone material is removed.

In an optional embodiment, the cutting areas may be situated over the entire proximal area of the blades and not only on the edge. Wherein teeth or protruding cutting elements and second protruding cutting elements may extend proximally from the proximal reamer area towards the bone to be reamed. The cutting elements preferably have distal leading through cutting openings and second distal leading through cutting openings which are extending distally from the proximal side of the cutting elements through the entire thickness of the reamer blade such that, the bone cut may be excised from the cutting section therethrough. The protruding cutting elements may be in an aligned or random array. The protruding cutting elements and second protruding cutting elements may have any geometrical form such as oval, triangular, square, diamond, or just any other shape suitable for being used as a blade for cutting the bone. Any other form of cutting blades may be used with the described reamer embodiment. Alternatively, sandpaper material (such as diamond sand) may be used as cutting elements for finer and more precise drilling operations.

A retro pin may be detachably attached to the reamer by means of a bayonet system. Preferably, it may be directly screwed in the proximal end of the reamer. Even more preferably, it may be secured with a clamp or by an array of fixing screws, or just by one or two screws.

Preferably, the shaft may be detachably secured in a ball which is contained in a socket at the center of the center connection ring of the reamer. The ball operates as a gimbal and allows the reamer the freedom of rotation in all three axes around the reamer shaft. This gives the operator the freedom to drill a hole at any angle. Connected to the gimbal are screws freely moving in slots in the side of the center connection ring in order to transmit the rotational force and direction from the retro pin shaft to the reamer. The reamer may penetrate more bone on one side than on the other due to the angle of the shaft, but not deeper than the depth limit stop allows. The reamer having a gimbal in connection with the depth stop may reach a perfect hole for an implant at the precise depth needed at the end of the surgical procedure.

A further aspect of the invention relates to a method for drilling a hole into a bone joint. When in use, the reamer is preferably connected to a retro pin from the front or by a shaft from the back. The reamer may be placed above the immediate exterior part of a bulbous area of the bone joint to be reamed. With the help of a rotating instrument, such as a drill attached to the retro pin or the shaft, the reamer rotates around its shaft's axis. The operator preferably applies slight pressure force against the drill when the reamer is connected at the back by a shaft or applies a pulling force in case of a reamer attached to a retro pin in the front. This force is in turn transmitted via the reamer retro pin or the shaft to the bone to be reamed. By applying a continuous and constant pressure, the front cutting section of the reamer cuts off parts of the bone.

Generally, the hardest part of a bone is the cortical part or the exterior part of a bone. Compared thereto, the interior of the bone, the cancellous part, has a spongy consistency which is a lot less dense than the exterior part. Because of the bulbous parabolic form of the bone joint the reamer starts first reaming off parts on the highest point of the bulb of the bone joint. The reamer connects the bone first with its center which is the sharpest part of the reamer. Gradually, more bone of the parabolic bulbous bone joint is shaved or cut off and more area of the bulbous part of the bone joint comes into contact with the blades of the reamer. At some point in time, depending of the shape, thickness, and density of the cortical part of the bone to be reamed, the bone is penetrated by the reamer's cutting area. At this point, conventional normal reamers with constant sharpness become uncontrollable and a shock or a jerky forward movement is observed. Normally, the operator exerts too much pressure on the drill and overshoots when the hard shell of the bone gives way. This is normally corrected by reducing the exerted pressure force upon the reamer. The reason for this jerky movement is that the same force is applied both to the large parabolic bulbous hard shell bone area and to the subsequent remaining cancellous or spongy part. Since the reamer blade sharpness is constant and at the same speed the reamer transits suddenly from the hard bone into the soft bone with a far too greater force. In this case the bone is cut instantaneously and the reamer penetrates the cancellous part of the bone before the operator can correct the speed of the drill or of the force applied.

This problem is overcome in the present embodiment by the variable sharpness of the cutting section. The blade is sharper at the center and duller gradually towards the edge of the reamer. Once the hardest exterior cortical part of the bulbous bone is penetrated by the reamer's sharper cutting section, the remaining bone shell continues to be cut at a slower intensity but with a similar speed and force applied by the operator. The sudden change in penetration is alleviated by the blunter exterior cutting section of the reamer blades. As such, the operator does not feel a sudden easiness in drilling and has at all time perfect control over the drilling procedure. He cannot overshoot or move the reamer in a non-axial direction due to a sudden penetration shock. The reamer may only penetrate the bulbous bone until the proximally offset circular depth limit stop comes into contact with the bone. This is preferably the maximum depth of the reamed hole possible and also the maximum radius of the cut off area.

Furthermore, it is preferred that the shaft or the retro pin are flexible or at least semi-rigid, or with at least one or more movable angular joints when connected directly to a reamer without the use of a gimbal. This would allow to bend the shaft at least once in one desired direction. The shaft may be adapted to any form and/or curve which is required to reach a specific position within the body, preferably at a bone or a joint.

If the end of the hole is reached, the drilling process may be stopped, for example by switching off a motor rotating the drill. After stopping the drill, this may be restarted again manually by pressing a button. The end of the hole may be detected by visually evaluating the distance between the depth limit stop and the bone.

Preferably, the materials used for the shaft and the reamer may be of titanium, titanium alloy, titanium-aluminum-vanadium, surgical stainless steel, martensitic steel, or any stainless steel or alloy thereof.

The instruments, systems and methods may be applied to a variety of fields, for example sports medicine, implant surgery, trauma, etc.

### Description of Drawings

In the following the invention will be described by way of example, without limitation of the general inventive concept, on examples of embodiment with reference to the drawings.
- Figure 1: shows a perspective view of a flat reamer with two blade wings, a gimbal, and a circular depth limit stop.
- Figure 2: shows a sectional lateral view along the horizontal reamer axis of a flat reamer.
- Figure 3: shows a lateral view of a flat reamer in the drilling position of bone with a retro pin.
- Figure 4: shows a lateral view of a flat reamer after drilling into a bone with a retro pin.
- Figure 5: shows a sectional lateral view of a flat reamer, bone, and retro pin in the drilling position.
- Figure 6: shows a sectional lateral view of a flat reamer, bone, and retro pin after drilling into a bone.
- Figure 7: shows a 3D view of a flat reamer with semi-circular depth limit stops after the drilling process.
- Figure 8: shows a view of the distal part of a flat reamer with two cutting blades and with semi-circular depth limit stops.
- Figure 9: shows a view of the distal part of a flat reamer with two cutting blades and with semi-circular depth limit stops, and the virtual tracks of the variable cutting blades.

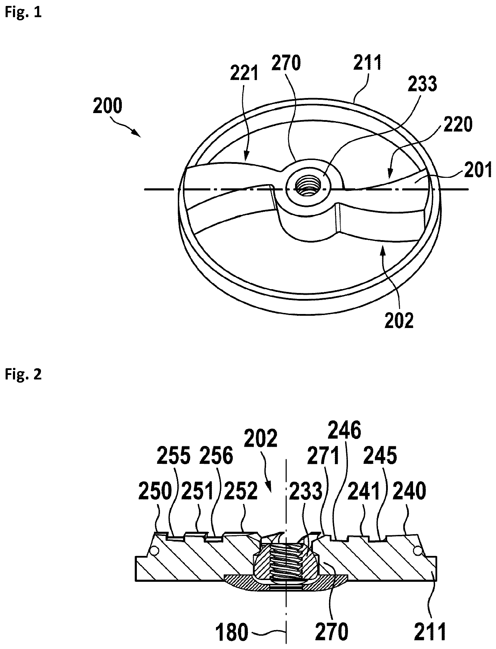

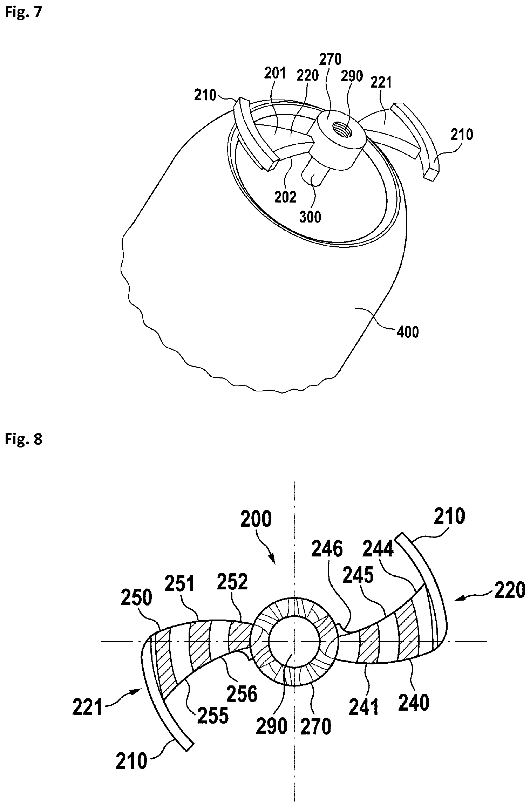

Figure 1 shows a flat reamer 200 having a distal area 201 and a proximal area 202. The reamer is pulled towards the user in order to effectuate the reaming procedure. The distal area 201 is passive and is further away from the operator. The active part incorporating cutting sections 240 - 241 and 250 - 252 is closer to the operator and is considered to be the proximal side 202 of the reamer 200. In this embodiment, the reamer 200 comprises a first 220 and a second 221 opposing blade wing centrally connected to a center connection ring 270, and externally connected to a circular depth limit stop 211. A reamer shaft 120 as shown in Fig. 7, a retro pin 300, or any other handles or tools may be permanently attached to the reamer or detachably attached through different connections such as screw and thread, or bayonet connector, or press fit connector, or any other connector. In the present Figure 1, the reamer shaft 120 or a retro pin 300 may be connected to a gimbal 233 positioned in the center of the flat reamer 200. The gimbal is preferably threaded for connecting a retro pin and may be loosely held within the connection ring by two gimbal screws (not shown) extending through two screw holes in the connection ring wall case. The gimbal screws may be placed opposite to each other and may be used to pass the rotational shaft force to the flat reamer 200 and the blade wings 220, 221. The optional wall screw holes 230 preferably are elongated from a distal to a proximal direction allowing the gimbal screws and therefore the gimbal 233 to move distally and proximally through the elongated wall screw hole 230 and the connection ring respectively. As such, the flat reamer 200 may move and oscillate in any direction even if the shaft or the retro pin is fixed on an axis. The flat reamer 200 rotates around the reamer axis 180 as shown in Figure 2. The at least one first blade wing 220 and the at least one second blade wing 221 have a first side and an opposing second side, both sides being connected to the depth limit stop 210, 211 and the center connection ring 270, wherein the first side and the second side may be parallel or form an acute angle at the center connection ring 270. In this embodiment, the opposing blade wings 220, 221 have a triangular geometrical shape with the two triangular sides slightly arched in the rotation direction. The triangular blades may be equilateral or isosceles or scalene. The hypotenuses 212, 213 of the triangular-shaped blade wings 220, 221 are arched and connect to the circular depth limit stop 211. The blade wings 220, 221 may be thicker than the circular depth limit stop 211 and may extend proximally away from the circular depth limit stop 211. The distance, in which the blades extend in the proximal direction starting from the circular depth limit stop 211 is the end deepness the reamer may penetrate a bone.

Figure 2 shows a sectional lateral view along the horizontal reamer axis 180 of a flat reamer. The distal area 201 of the flat reamer 200 is almost flat in the Figure, with a small through hole 290 to accommodate the head of a retro pin distal plate, wherein the proximal area 202 shows the cutting sections 240 - 241 and 250 - 252. In this example, the cutting sections are intermittently separated by channels or recesses 245 - 246 and 255 - 256. The cutting sections 241 and 252 closer to the center of the reamer may be very sharp, wherein the more exterior cutting sections 240 and 250 may be duller. The center of the reamer has a center connection ring 270 which may also have cutting sections. In this particular embodiment the cutting sections from the first blade wing run radially adjacent to the cutting section of the second blade wing. In a preferred embodiment, the cutting sections may not necessarily run radially adjacent to the other cutting section of another blade wing, but may have some space. In another preferred embodiment, the cutting sections may not necessarily run radially adjacent to the other cutting section of another blade wing, but these may also overlap up to 100 %. The cutting sections may also be wider on one blade wing and narrower on the other such that the wider cutting section overlaps to more than 100 % the radial path of the narrow cutting section. A narrow and a wider cutting section are beneficial in some surgery procedure. The narrow cutting section may be sharper and may cut first into the bone, wherein the wider cutting section shaves off a larger area thereafter.

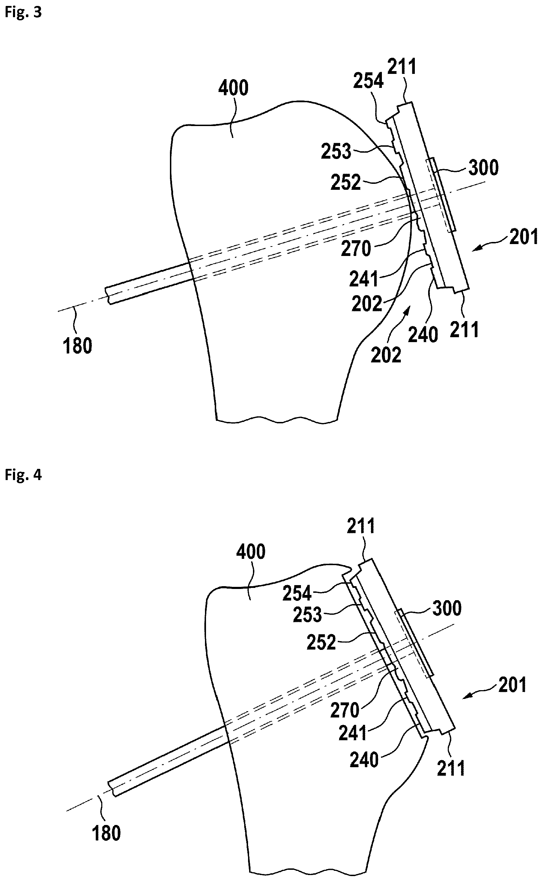

Figure 3 shows a lateral view of a flat reamer in the drilling position of a bone 400 with a retro pin. In some surgery procedures, it is more advantageous to work with a retro pin rather than a pressing shaft. In this particular example, the retro pin 300 has first been inserted through the joint and the flat reamer 200 has been attached to the pin. By applying a pulling force to the retro pin 300, the cutting sections 240 - 241 and 250 - 252 of the flat reamer 200 engage the bulbous area of the bone to be reamed. The sharpest cutting sections 252 and 241 contact the bone 400 first. The least sharp cutting sections 240 and 254 contact the bone 400 last. As such a change in the force with which the operator operates the surgical drill is not needed. Once the top part of the bone has been penetrated, the lateral bone walls will also be cut off at the same speed but with duller blades. The flat reamer 200 protrudes the bone until the depth limit stop 211 limits any further bone penetration by sliding on the edge of the hole created by the flat reamer 200. As the depth limit stop 211 has no cutting sections, it cannot cut the bone and penetrate into the bone.

Figure 4 shows a lateral view of a flat reamer after drilling into a bone with a retro pin. The depth is dictated by the height of the flat reamer which is up to the circular depth limit stop 211.

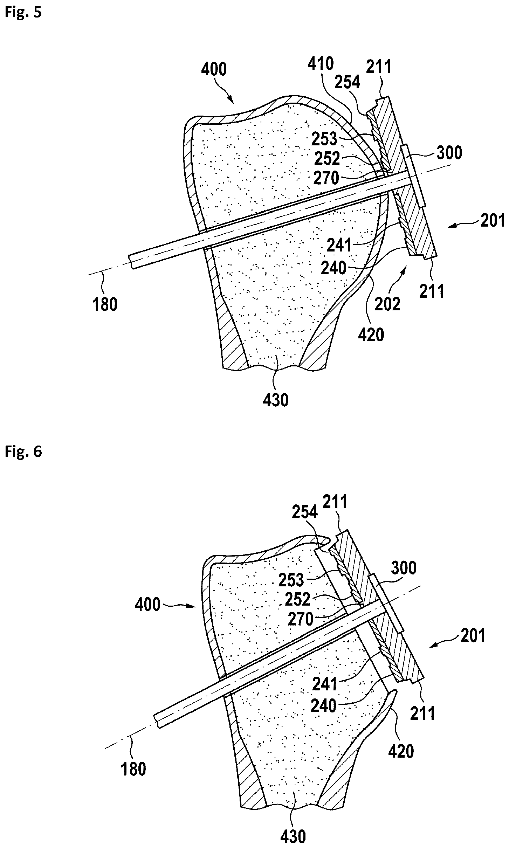

Figure 5 shows a sectional lateral view of a flat reamer 200, a bone 400, and a retro pin 300 in the drilling position. By the sectional view a better understanding can be achieved of the surgical process. For instance, the harder exterior cortical part 420 of the bone can be clearly illustrated. The flat reamer 200 in this Figure was placed above the bulbous part 410 of the bone joint to be reamed.

Figure 6 shows a sectional lateral view of a flat reamer 200, bone 400, and a retro pin 300 after drilling into a bone 400 took place. After the surgical step, the flat reamer protruded the exterior cortical part 420 of the bone and entered the spongy or cancellous 430 bone part. The same could be achieved also with a reamer with an external shaft. It may also be possible to use a normal reamer shaft and press onto the reamer to drill the hole rather than pull onto a retro pin. The remaining cancellous 430 bone part is in such a case untouched since there is no through-hole due to the retro pin, and therefore it would form an ideal area to place a new implant.

Figure 7 shows a 3D view of a flat reamer 200 with two arc segment depth limit stops 210 after the drilling process. The flat reamer 200 may not incorporate an entire circular depth limit stop 211 but rather have only a couple of short arc segments depth limit stops 210. This is a lighter version of the flat reamer 200. The semi-circular depth limit stops 210 create a similar circular appearance when rotating as with the circular depth limit stop 211 and also delimit efficiently the depth of the hole.

Figure 8 shows a view of the proximal or front area 202 of a flat reamer 200 with two cutting blade wings 220, 221 and with an arc segment depth limit stops 210. The flat reamer 200 with two cutting blade wings 220, 221 and with the arc segment depth limit stops 210 allows for a wider viewing area during surgery, since the circular limit stop 211 is not in the way.

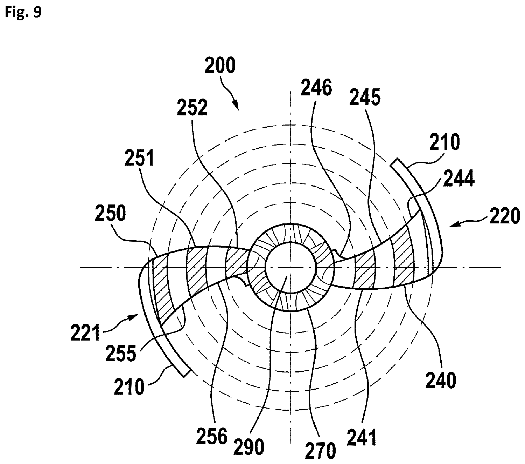

Figure 9 shows the reamer of Figure 8 together with the tracks (dashed) in which the individual cutting sections are running and removing bone material. As shown, the tracks are all concentrical and neighbored to each other, such that a complete circular bone area can be removed. Preferably, there is some overlap, most preferably in a range between 10% and 50% between the tracks, such that all bone material is removed.

The systems and methods of the present invention enable a single surgeon to operate in a safe and precise environment, to target an area of interest, and to obtain the desired results without any complications such as overshooting or injuring neighboring tissue.

### List of reference numerals

- 200: flat reamer
- 201: distal area
- 202: proximal area
- 210: arc segment depth limit stop
- 211: circular depth limit stop
- 212-213: hypotenuses
- 220: one first blade wing
- 221: one second blade wing
- 230: wall screw hole
- 233: gimbal
- 240-241: first cutting sections
- 245-246: first recesses
- 250-252: second cutting sections
- 255-256: second recesses
- 267-269: distal through cutting openings
- 270: center connection ring
- 271: cutting sections
- 280: vertical shaft axis
- 287-289: second distal through cutting openings
- 290: through-hole
- 300: retro pin
- 400: bone
- 410: bulbous part
- 420: cortical bone
- 430: cancellous bone

## Claims

1. A reamer (200) extending between a planar proximal reamer area (202) and a planar distal reamer area (201) comprising:
a center connection ring (270) configured for attachment of a retro pin (300),
at least one first blade wing (220) and at least one equally shaped second blade wing (221) extending between said planar areas (201, 202) from the center connection ring (270) radially towards a depth limit stop (210, 211), the at least one first blade wing (220) and the at least one second blade wing (221) having at the proximal reamer area (202) first cutting sections (240-241) and second cutting sections (250-252) intermittently separated by first recesses (245-246) and second recesses (255-256),
**characterized in that**,
the cutting sections are sharpest on or adjacent to the central connection ring (270) of the reamer and gradually decrease in sharpness with the distance away from said connection ring (270).

2. The reamer (200) according to claim 1,
**characterized in that**,
the at least one first blade wing (220) and the at least one second blade wing (221) have a first side with an arched shape in the rotational direction and an opposing second side, both sides being connected to the depth limit stops (210, 211) and the center connection ring (270), wherein the first side and the second side are parallel or form an acute angle at the center connection ring (270).

3. The reamer (200) according to any one of the preceding claims,
**characterized in that**,
said cutting sections comprise single blades situated on the forward rotating edge of said proximal area of said first and second blade wings (220, 221).

4. The reamer (200) according any one of the preceding claims,
**characterized in that**,
the radial distance of the cutting sections (240 - 241, 250 - 252) of one blade wing equals the radial distance of the recesses (245 - 246, 255 - 256) on the opposite blade wing.

5. The reamer (200) according any one of the preceding claims,
**characterized in that**,
the radial width of the cutting sections (240 - 241, 250 - 252) of one blade wing is larger than the radial width of the recesses (245 - 246, 255 - 256) on the opposite blade wing.

6. The reamer (200) according to any one of the preceding claims,
**characterized in that**,
the retro pin (300) is detachably attached to the reamer (200) by means of a bayonet system or is directly screwed in the proximal end of the reamer or is secured with a clamp or by an array of fixing screws or just one or two screws.

7. The reamer (200) according to any one of the preceding claims,
**characterized in that**,
the retro pin (300) may be detachably secured in a gimbal (233) contained in a socket at the center of the reamer wherein the reamer is connected to said gimbal suitable for transmitting the rotational force and direction from the retro pin (300) to the reamer, wherein the gimbal (233) allows the reamer the freedom of movement in all three axes around the retro pin (300).

8. The reamer (200) according to any one of the preceding claims,
**characterized in that**,
the blade wings (220, 221) may have different thickness from 0,1 mm - 200 mm or from 0,1 mm - 20 mm or from 0,1 mm - 10 mm.

9. The reamer (200) according to any one of the preceding claims,
**characterized in that**,
the blade wings (220, 221) may have different radii from 10 mm - 100 mm, or from 20 mm -70 mm or from 25 mm - 50 mm.

10. The reamer (100, 200) according to any one of the preceding claims,
**characterized in that**,
the materials used for the shaft and the reamer head comprise at least one of titanium, titanium alloy, titanium-aluminum-vanadium, surgical stainless steel, martensitic steel, or any alloy or combination thereof.

## Patentansprüche

1. Fräswerkzeug (200), sich zwischen einem planaren proximalen Fräswerkzeugbereich (202) und einem planaren distalen Fräswerkzeugbereich (201) erstreckend, Folgendes umfassend:
einen mittleren Verbindungsring (270), der zur Befestigung eines rückwärtsgerichteten Stifts (300) ausgebildet ist, mindestens einen ersten Messerflügel (220) und mindestens einen gleich geformten zweiten Messerflügel (221), die sich zwischen den planaren Bereichen (201, 202) vom mittleren Verbindungsring (270) radial zu einem Tiefenbegrenzungsanschlag (210, 211) erstrecken, wobei der mindestens eine erste Messerflügel (220) und der mindestens eine zweite Messerflügel (221) am proximalen Fräswerkzeugbereich (202) erste Schneidabschnitte (240-241) und zweite Schneidabschnitte (250-252) aufweisen, die intermittierend durch erste Aussparungen (245-246) und zweite Aussparungen (255-256) getrennt sind,
**dadurch gekennzeichnet, dass**
die Schneidabschnitte auf oder angrenzend am mittleren Verbindungsring (270) des Fräswerkzeugs am schärfsten sind und mit dem Abstand vom Verbindungsring (270) schrittweise an Schärfe abnehmen.

2. Fräswerkzeug (200) nach Anspruch 1,
**dadurch gekennzeichnet, dass**
der mindestens eine erste Messerflügel (220) und der mindestens eine zweite Messerflügel (221) eine erste Seite mit einer Bogenform in der Drehrichtung und eine gegenüberliegende zweite Seite aufweisen, wobei beide Seiten mit den Tiefenbegrenzungsanschlägen (210, 211) und dem mittleren Verbindungsring (270) verbunden sind, wobei die erste Seite und die zweite Seite parallel verlaufen oder einen spitzen Winkel am mittleren Verbindungsring (270) bilden.

3. Fräswerkzeug (200) nach einem der vorhergehenden Ansprüche,
**dadurch gekennzeichnet, dass**
die Schneidabschnitte einzelne Messer aufweisen, die sich auf der vorwärts drehenden Kante des proximalen Bereichs des ersten und zweiten Messerflügels (220, 221) befinden.

4. Fräswerkzeug (200) nach einem der vorhergehenden Ansprüche,
**dadurch gekennzeichnet, dass**
der radiale Abstand der Schneidabschnitte (240-241, 250-252) eines Messerflügels dem radialen Abstand der Aussparungen (245-246, 255-256) auf dem gegenüberliegenden Messerflügel entspricht.

5. Fräswerkzeug (200) nach einem der vorhergehenden Ansprüche,
**dadurch gekennzeichnet, dass**
die radiale Breite der Schneidabschnitte (240-241, 250-252) eines Messerflügels größer ist als die radiale Breite der Aussparungen (245-246, 255-256) auf dem gegenüberliegenden Messerflügel.

6. Fräswerkzeug (200) nach einem der vorhergehenden Ansprüche,
**dadurch gekennzeichnet, dass**
der rückwärtsgerichtete Stift (300) mittels eines Bajonettsystems lösbar am Fräswerkzeug (200) befestigt oder direkt im proximalen Ende des Fräswerkzeugs eingeschraubt oder mit einer Klemme oder durch eine Anordnung von Befestigungsschrauben oder nur eine oder zwei Schrauben befestigt ist.

7. Fräswerkzeug (200) nach einem der vorhergehenden Ansprüche,
**dadurch gekennzeichnet, dass**
der rückwärtsgerichtete Stift (300) lösbar in einem Kardanring (233) befestigt werden kann, der in einer Buchse in der Mitte des Fräswerkzeugs enthalten ist, wobei das Fräswerkzeug mit dem Kardanring verbunden ist, der zur Übertragung der Drehkraft und Richtung vom rückwärtsgerichteten Stift (300) auf das Fräswerkzeug geeignet ist, wobei der Kardanring (233) dem Fräswerkzeug Bewegungsfreiheit in allen drei Achsen um den rückwärtsgerichteten Stift (300) erlaubt.

8. Fräswerkzeug (200) nach einem der vorhergehenden Ansprüche,
**dadurch gekennzeichnet, dass**
die Messerflügel (220, 221) eine unterschiedliche Dicke von 0,1 mm-200 mm oder von 0,1 mm-20 mm oder von 0,1 mm-10 mm aufweisen können.

9. Fräswerkzeug (200) nach einem der vorhergehenden Ansprüche,
**dadurch gekennzeichnet, dass**
die Messerflügel (220, 221) unterschiedliche Radien von 10 mm-100 mm oder von 20 mm-70 mm oder von 25 mm-50 mm aufweisen können.

10. Fräswerkzeug (100, 200) nach einem der vorhergehenden Ansprüche,
**dadurch gekennzeichnet, dass**
die für die Welle und den Fräswerkzeugkopf verwendeten Materialien mindestens eines aus Titan, Titanlegierung, Titan-Aluminium-Vanadium, chirurgischem rostfreien Stahl, martensitischem Stahl oder irgendeiner Legierung oder Kombination davon umfasst.

## Revendications

1. Alésoir (200) s'étendant entre une zone d'alésoir proximale plane (202) et une zone d'alésoir distale plane (201) comprenant :
une bague de connexion centrale (270) configurée pour la fixation d'une broche rétro (300),
au moins une première aile de lame (220) et au moins une deuxième aile de lame de forme identique (221) s'étendant entre lesdites zones planes (201, 202) à partir de la bague de connexion centrale (270) radialement vers une butée de limite de profondeur (210, 211), l'au moins une première aile de lame (220) et l'au moins une deuxième aile de lame (221) ayant au niveau de la zone d'alésoir proximale (202) des premières sections de coupe (240-241) et des deuxièmes sections de coupe (250-252) séparées par intermittence par des premiers évidements (245-246) et des deuxièmes évidements (255-256),
**caractérisé en ce que**,
les sections de coupe sont très serrées sur ou adjacentes à la bague de connexion centrale (270) de l'alésoir et diminuent progressivement en resserrement avec l'éloignement de ladite bague de raccordement (270).

2. Alésoir (200) selon la revendication 1,
**caractérisé en ce que**,
l'au moins une première aile de lame (220) et l'au moins une deuxième aile de lame (221) ont un premier côté avec une forme arquée dans la direction de rotation et un deuxième côté opposé, les deux côtés étant connectés aux butées de limite de profondeur (210, 211) et à la bague de connexion centrale (270), dans lequel le premier côté et le deuxième côté sont parallèles ou forment un angle aigu au niveau de la bague de connexion centrale (270).

3. Alésoir (200) selon l'une quelconque des revendications précédentes,
**caractérisé en ce que**,
lesdites sections de coupe comprennent des lames uniques situées sur le bord de rotation avant de ladite zone proximale desdites première et deuxième ailes de lame (220, 221) .

4. Alésoir (200) selon l'une quelconque des revendications précédentes,
**caractérisé en ce que**,
la distance radiale des sections de coupe (240 - 241, 250 - 252) d'une aile de lame est égale à la distance radiale des évidements (245 - 246, 255 - 256) sur l'aile de lame opposée.

5. Alésoir (200) selon l'une quelconque des revendications précédentes,
**caractérisé en ce que**,
la largeur radiale des sections de coupe (240 - 241, 250 - 252) d'une aile de lame est plus large que la largeur radiale des évidements (245 - 246, 255 - 256) sur l'aile de lame opposée.

6. Alésoir (200) selon l'une quelconque des revendications précédentes,
**caractérisé en ce que**,
la broche rétro (300) est fixée de manière amovible à l'alésoir (200) au moyen d'un système à baïonnette ou est directement vissée à l'extrémité proximale de l'alésoir ou est fixée à l'aide d'une pince ou d'un ensemble de vis de fixation ou juste d'une ou deux vis.

7. Alésoir (200) selon l'une quelconque des revendications précédentes,
**caractérisé en ce que**,
la broche rétro (300) peut être fixée de manière amovible dans une cardan (233) contenu dans une douille au centre de l'alésoir, dans lequel l'alésoir est relié audit cardan utilisable pour la transmission de la force de rotation et de la direction à partir de la broche rétro (300) à l'alésoir, dans lequel le cardan (233) permet à l'alésoir la liberté de mouvement dans les trois axes autour de la broche rétro (300).

8. Alésoir (200) selon l'une quelconque des revendications précédentes,
**caractérisé en ce que**,
les ailes de lame (220, 221) peuvent avoir une épaisseur différente de 0,1 mm - 200 mm ou de 0,1 mm - 20 mm ou de 0,1 mm à -10 mm.

9. Alésoir (200) selon l'une quelconque des revendications précédentes,
**caractérisé en ce que**,
les ailes de lame (220, 221) peuvent avoir des rayons différents de 10 mm - 100 mm, ou de 20 mm -70 mm ou de 25 mm-50 mm.

10. Alésoir (100, 200) selon l'une quelconque des revendications précédentes,
**caractérisé en ce que**,
les matériaux utilisés pour l'arbre et la tête d'alésoir comprennent au moins l'un de titane, d'alliage de titane, de titane-aluminium-vanadium, d'acier inoxydable chirurgical, d'acier martensitique, ou de l'un quelconque alliage ou d'une combinaison de ceux-ci.
